**Europäisches Patentamt**

**European Patent Office**

**Office européen des brevets**

(19)

(11) Veröffentlichungsnummer: **0 482 294 A2**

## EUROPÄISCHE PATENTANMELDUNG

(12)

(21) Anmeldenummer: **91110034.5**

(22) Anmeldetag: **19.06.91**

(51) Int. Cl.$^5$: **G01N 33/20**

(30) Priorität: **26.10.90 DE 4034098**

(43) Veröffentlichungstag der Anmeldung:
**29.04.92 Patentblatt 92/18**

(84) Benannte Vertragsstaaten:
**ES FR GB IT**

(71) Anmelder: **AEG-Elotherm GmbH**
**Hammesberger Strasse 31**
**W-5630 Remscheid-Hasten(DE)**

(72) Erfinder: **Stengel, Edgar**
**Erdelenstrasse 35**
**W-5630 Remscheid(DE)**
Erfinder: **Reinke, Friedhelm, Prof. Dr.-Ing.**
**Oelingrath 19**
**W-5630 Remscheid(DE)**

(74) Vertreter: **Patentanwaltsbüro Cohausz &**
**Florack**
**Schumannstrasse 97**
**W-4000 Düsseldorf 1(DE)**

(54) **Verfahren zur Kontrolle der Güte von gehärteten Wellen, insbesondere Kurbelwellen.**

(57) Die Erfindung betrifft ein Verfahren zur Kontrolle der Güte von Wellen, insbesondere Kurbelwellen, nach einer insbesondere durch elektroinduktive Erwärmung und anschließenden Abschreckung erzeugten Randschichthärtung.

Kennzeichen der Erfindung ist, daß als Maß für die Güte der Einhärtung die Längenänderung der Welle nach der Aufwärmphase und die nach der Abschreckphase gemessen und anhand der Abweichung von einem vorgegebenen Sollwert die weitere Verwendbarkeit der Welle festgelegt wird.

Die Erfindung betrifft ein Verfahren zur Kontrolle der Güte von Wellen, insbesondere Kurbelwellen, nach einer Randschichthärtung, insbesondere durch elektroinduktive Erwärmung und anschließende Abschreckung.

Für die beim Härten von Wellen mögliche Einhärtetiefe sind die folgenden Prozeßdaten von besonderer Bedeutung:
- Wärmezufuhr
- Erwärmungszeit
- Härtetemperatur
- Abschreckmitteltemperatur
- Abschreckmittelmenge
- Abschreckzeit

Nur bei Einhaltung genauer Vorgaben für diese Parameter lassen sich gehärtete Wellen herstellen, die alle Anforderungen hinsichtlich der Lebensdauer erfüllen.

Bisher hilft man sich zur Qualltätssicherung damit, alle Prozeßdaten einzeln zu überwachen. Es handelt sich dabei um eine Vorwärtskontrolle. Trotzdem verbleibt der Ergebniskontrolle, d.h. der Überwachung der beim Härtungsprozeß erzielten Einhärtetiefe, in der Praxis eine besondere Bedeutung.

Um diese Kontrolle durchzuführen, war es bisher nötig, gehärtete Wellen stichprobenartig aus dem Fertigungsprozeß herauszunehmen und Schliffbilder und Härteverlaufskurven anzufertigen. Oft wird die Produktion während dieser Untersuchungen stillgesetzt. Diese Prüfverfahren sind besonders zeit- und kostenaufwendig und nur unter Zerstörung der geprüften Welle durchführbar. Auf diese Weise ist es insbesondere nicht möglich jede Welle zu überprüfen.

Aufgrund der gestiegenen Anforderungen an die Qualität des Bauteils, insbesondere bei dem heute sehr hohen Automatisierungsgrad der Fertigung, ist es aber gerade erforderlich, für jede einzelne Welle ein zerstörungsfrei zu gewinnendes Merkmal zu finden, das die Härtung eindeutig charakterisiert.

Daher besteht die Aufgabe der Erfindung darin, ein Verfahren anzugeben, mit dem es auf einfache Weise möglich ist, jede Welle nach der Härtung zerstörungsfrei auf die erzielte Einhärtetiefe und damit auf die Güte hin zu überprüfen.

Diese Aufgabe wird erfindungsgemäß dadurch gelöst, daß als Maß für die Güte der Einhärtung die Längenänderung der Welle nach der Aufwärmphase und die nach der Abschreckphase gemessen und anhand der Abweichung der Meßwerte von einem vorgegebenen Sollwert die weitere Verwendbarkeit der Welle festgestellt wird.

So erhält man für jede einzelne Welle nach dem Härten einen Meßwert, der direkt in Beziehung zur Qualität der erreichten Einhärtung steht. Dazu müssen lediglich zwei Längenmessungen an jeder Welle durchgeführt werden. Dies kann ohne Beschädigung der Welle geschehen. Die Längenmessung und die Bestimmung der Längenabweichung von einem vorgegebenen Sollwert mit herkömmlichen Meß- und Auswertmitteln leicht automatisch durchgeführt werden. Darüber hinaus ist es nun auf einfache Weise möglich, die anhand der Auswertung der Meßergebnisse die Entscheidung über die weitere Verwendbarkeit der Welle zu treffen.

Genauso lassen sich aus dem Vergleich von vorgegebenem Soll- und gemessenem Istwert die Bedingungen für die nachfolgende Härtung einer Welle verändern.

Im folgenden soll das erfindungsgemäße Verfahren an einem Beispiel näher erläutert werden.

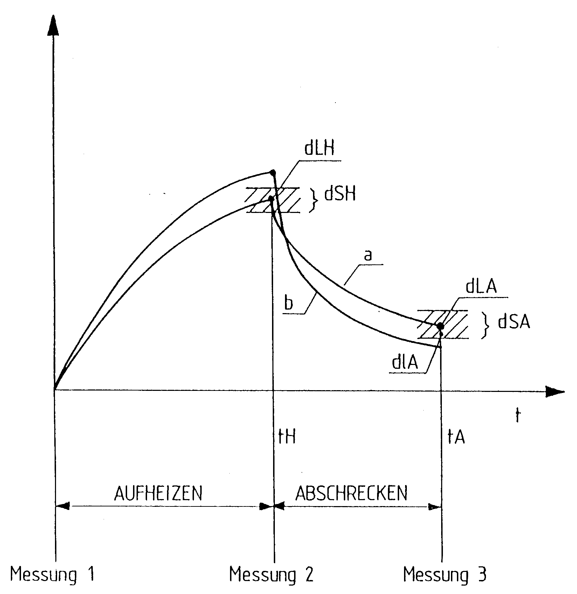

Die Figur zeigt ein Diagramm, in welchem der ideale Verlauf a) der Längenänderung einer Welle beim Aufheizen und Abschrecken, der Toleranzbereich der zulässigen Abweichungen nach dem Aufheizende dSH und dem Abschreckende dSA und der Kurvenverlauf b) der Längenänderung einer fehlerhaft gehärteten Welle aufgetragen sind.

Während der Erwärmung kann die Längenänderung der Welle beispielsweise durch ein optisches, interementales, induktives oder piezo-resistives Längenmeßverfahren ständig überwacht werden. Nach der Aufwärmzeit tH wird eine erste Messung der Längenänderung dLH der Welle ausgewertet.

Nach Beendigung der Härtung wird die Längenänderung dLA der Welle ausgewertet und mit den Toleranzbändern dSH bzw. dSA verglichen. Liegt das Ergebnis im Toleranzbereich dSA des Sollwertes, so ist eine ausreichende Einhärtetiefe erreicht worden, anderenfalls muß die Welle ausgesondert werden. Wird der Toleranzbereich dSH des Sollwertes dLH überschritten, kann die zugeführte Wärmemenge entsprechend bei nachfolgenden Härtungen verändert werden. Entsprechendes gilt für die Abschreckung. - Unregelmäßigkeiten im zeitlichen Verlauf erlauben darüber hinaus Rückschlüsse auf die Ursache.

**Patentansprüche**

1.  Verfahren zur Kontrolle der Güte von Wellen, insbesondere Kurbelwellen, nach einer insbesondere durch elektroinduktive Erwärmung und anschließenden Abschreckung erzeugten Randschichthärtung,
    **dadurch gekennzeichnet,** daß als Maß für die Güte der Einhärtung die Längenänderung der Welle nach der Aufwärmphase und die nach der Abschreckphase gemessen und anhand der Abweichung von einem vorgegebenen Sollwert die weitere Verwendbarkeit der Welle festgelegt wird.

2. Verfahren nach Anspruch 1,
**dadurch gekennzeichnet,** daß die Härtebedingungen in Abhängigkeit von der ermittelten Sollwert-Abweichung verändert wird.